# EUROPEAN PATENT APPLICATION

(11) **EP 2 060 228 A1**
(43) Date of publication of application: **20.05.2009**
(21) Application number: 07763989.6
(22) Date of filing: 05.07.2007
(51) Int. Cl.: A61B 5/01, A61B 5/16

(54) **MEDICAL DEVICE WITH AN EMOTION MEASURING FINGER SHEATHING MEANS INCORPORATING WITH A LONG-DISTANCE CONTROL**

(30) Priority: 04.09.2006 CN 200620138766 U
(71) Applicant: Zhang, Fenglin, Tainan City, Taiwan (CN)
(72) Inventor: Zhang, Fenglin, Tainan City, Taiwan (CN)
(74) Representative: Metz, Paul
(86) International application number: PCT/CN2007/002077
(87) International publication number: WO 2008/028391

(57) **Abstract**

A medical system formed of an emotion measuring finger cot unit **(1)** and a remote control device **(2)** is disclosed. The emotion measuring finger cot unit **(1)** includes a finger cot **(14)** wearable on a finger of a person, a temperature sensor **(11)** mounted in the finger cot **(14)** for measuring the skin temperature of the finger, a processor **(12)** for computing the finger skin temperature signal obtained from the temperature sensor **(11),** and a wireless transmitter and receiver module **(13)** for transmitting the finger temperature data to the remote control device **(2)** wirelessly. The remote control device **(2)** can be a cell phone, multimedia player, PDA or computer, comprising a wireless transmitter and receiver module **(21)** for communication with the wireless transmitter and receiver module **(13)** of the emotion measuring finger cot unit **(1),** a control unit **(22)** electrically connected with the wireless transmitter and receiver module **(21),** and an input/output unit **(23)** electrically connected to the control unit **(22)** and operable by a person to input settings into the control unit **(22)** for controlling the measuring operation of the emotion measuring finger cot unit **(1).**

## Description

### BACKGROUND OF THE INVENTION

### 1. Field of the Invention

The present invention relates to a medical system and more particularly, to a medical system comprised of an emotion measuring finger cot unit and a remote control device. The medical system utilizes a temperature sensor in a finger cot to detect the skin temperature of the finger wearing the finger cot, for enabling the measured skin temperature signal to be processed through a processor and then transmitted to the remote control device wirelessly so that a medical practitioner can monitor the emotional status of the person wearing the emotion measuring finger cot unit and employ a therapy at a remote site.

### 2. Description of the Related Art

In medical practice, the surface temperature of human fingers may be measured to provide an index indicative of the emotion or stress response so that a further necessary step can be taken to make an adjustment or to overcome the problem.

The inventor of the present invention had invented, several years ago, many devices for measuring finger temperature. These devices are seen in U.S. Pat. No. 5,813,866, entitled "Finger temperature indicating ring"; U.S. Pat. No. 6,846,106B1, entitled "Finger temperature detecting device with relaxation indication". These designs commonly have a temperature sensor mounted in a carrier means wearable on a finger of a wearer to measure the temperature of a fixed survey area of the skin of the finger wearing the carrier means, enabling the measured data to be indicated through an indicating element

However, following fast development of technology and medical technique. The aforesaid designs cannot satisfy the requirements. Further, because the carrier means is provided with a face panel that carries a number of functional selection buttons, it has a certain dimension. When wearing the finger temperature indicating ring or finger temperature detecting device, the finger bears a weight and feels uncomfortable. Because of big size, the finger temperature indicating ring or finger temperature detecting device is not suitable for wearing on a finger of a young person.

Further, due to space limitation, the face panel of the carrier means does not allow installation of a big number of functional selection buttons. Therefore, the user may need to control the clicking time or to press two or more functional selection buttons at a time in order to achieve a particular function. Further, according to the aforesaid prior art designs, multiple functional lines may be connected to one same functional selection button through switch means, complicating the circuit layout and the operation and shortening the service life of the device.

Further, according to the aforesaid prior art designs, connecting the finger temperature indicating ring or finger temperature detecting device to a remote computer or other equipment requires the use of a cable. After connection of the finger temperature indicating ring or finger temperature detecting device to a remote computer or other equipment through a cable, the wearer must operate the functional selection buttons so that the measured temperature data can be transmitted to the connected remote computer or other equipment. Due to the limitations of the length of the cable and the number of functional selection buttons, the aforesaid prior art designs are unfriendly to the user.

Therefore, it is desirable to provide a medial system that eliminates the aforesaid drawbacks.

### SUMMARY OF THE INVENTION

The present invention has been accomplished under the circumstances in view. It is one object of the present invention to provide a medical system, which detects the skin temperature of a finger of a wearer and transmits the measured temperature data to a remote control unit wirelessly for recording and monitoring, for enabling a remote medical practitioner to give an emotional therapy in helping the wearer relieve stress. It is another object of the present invention to provide a medical system, which is comprised of a compact emotion measuring finger cot unit wearable on a finger of a person comfortably to measure the skin temperature of the finger, and a remote control device for communication with the emotion measuring finger cot unit wirelessly to monitor the measurement and to control the settings of the measurement (such as settings of the timer and standard time).

To achieve these and other objects of the present invention, the medical system is comprised of an emotion measuring finger cot unit and a remote control device. The motion measuring finger cot unit comprises a finger cot wearable on a finger of a person, a temperature sensor mounted in the finger cot and adapted for measuring the skin temperature of the finger wearing the finger cot, a processor electrically connected with the temperature sensor and adapted for computing the finger skin temperature signal obtained through the temperature sensor, and a wireless transmitter and receiver module electrically connected to the processor and controllable by the processor to transmit the finger temperature data to the remote control device wirelessly. The remote control device can be a cell phone, multimedia player, PDA or computer, comprising a wireless transmitter and receiver module for communication with the wireless transmitter and receiver module of the emotion measuring finger cot unit, a control unit electrically connected with the wireless transmitter and receiver module of the remote control device, and an input/output unit electrically connected to the control unit and operable by a person to input settings into the control unit or to modify data in the control unit.

Subject to the aforesaid arrangement, the medical system of the present invention has the following features and advantages:
1. The emotion measuring finger cot unit simply has a temperature sensor, a processor and a wireless transmitter and receiver module built in a finger cot without any face panel or functional selection buttons, therefore the emotion measuring finger cot unit has a compact size convenient for wearing on a finger of a person.
2. The invention has a wireless transmitter and receiver module, a control unit and an input/output unit be incorporated in a remote control device for inter-communication with the emotion measuring finger cot unit wirelessly, facilitating operation and avoiding errors.
3. Unlike functional limitation of the aforesaid prior art designs due to the limited size of the face panel, the control unit of the remote control device of the medical system of the present invention allows the user or medical practitioner to set or change the settings (settings of the timer or standard time, and other functions including delete, store, and etc.) of the program to be executed, having the control of the message obtained from the emotion measuring finger cot unit be provided in a human friendly manner so that the wearer or medical practitioner can easily known the physical/mental status of the wearer and the medical practitioner can give a professional diagnosis and therapy.
4. The medical system of the present invention has a simple structure. By means of wireless transmission and remote monitoring, a close interaction between the wearer and the practitioner can be achieved, improving treatment effect.

Further, the medical system can be connected to a remote communication system and server through the Internet so that a medical practitioner can obtain the measured skin temperature data from the emotion measuring finger cot unit through the remote control device conveniently at a remote site for on-line emotional stress management and therapy.

### BRIEF DESCRIPTION OF THE DRAWINGS

FIG. 1 is an elevational view of a medical system in accordance with the present invention.
FIG. 2 is a flow chart of the present invention.
FIG. 3 is a schematic drawing of the present invention, showing the emotion measuring finger cot unit worn on a finger of a wearer.
FIG. 4 is a schematic drawing showing the skin temperature measuring operation of the present invention.

### DETAILED DESCRIPTION OF THE PREFERRED EMBODIMENT

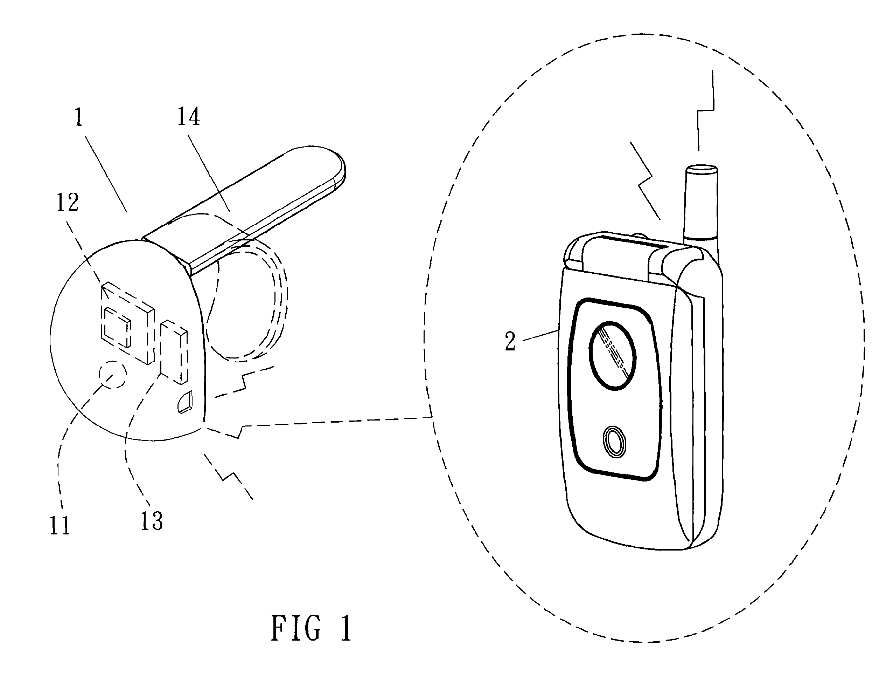

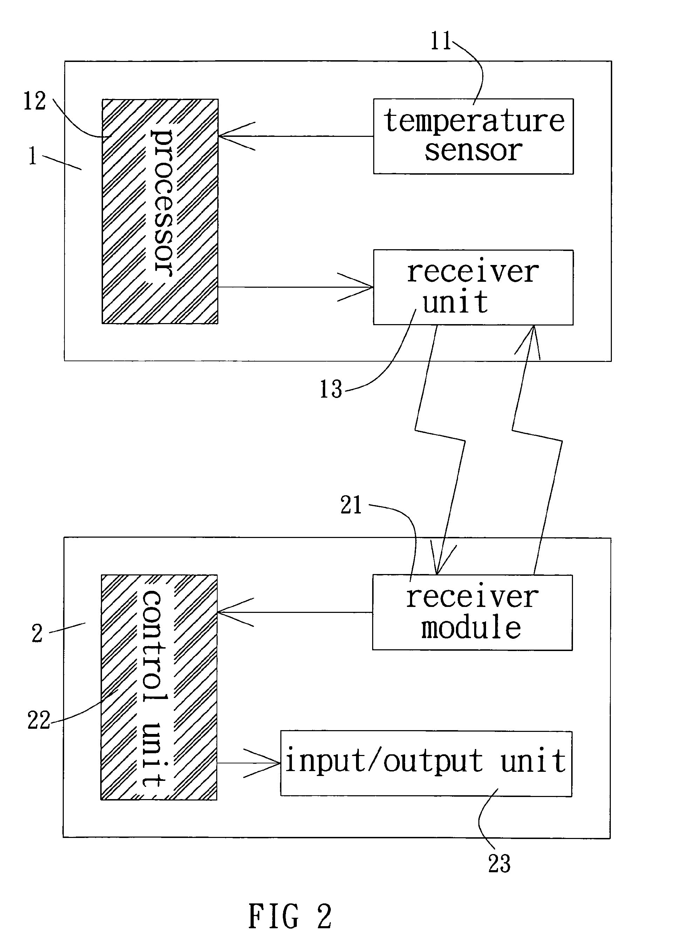

Referring to FIGS. 1 and 2, a medical system in accordance with the present invention is shown comprising an emotion measuring finger cot unit **1** and a remote control device **2.**

The emotion measuring finger cot unit **1** is a compact device having internal means and external means. The external means constitute the outer appearance of the emotion measuring finger cot unit **1.** According to this embodiment, the external means is made in the form of a finger cot **14.** The internal means is embedded in the external means, i.e., the finger cot **14,** comprising a temperature sensor **11** for measuring the skin temperature of a finger of a wearer, a processor **12** and a wireless transmitter and receiver module **13.** The temperature sensor **11** has the detecting surface thereof exposed to the outside of the finger cot **14** and kept in contact with the skin of the wearer's finger that wears the finger cot **14.** The processor **12** has data storage means built therein. Further, the processor **12** is electrically connected with the temperature sensor **12** and adapted for computing the finger skin temperature signal obtained through the temperature sensor **12** and storing the computed finger skin temperature data in the data storage means. The wireless transmitter and receiver module **13** is electrically connected to the processor **12,** and adapted for transmitting the finger temperature data to the remote control device **2** wirelessly. The finger cot **14** is a flexible member bendable to adjust its wearing tightness relative to the wearer's finger.

The remote control device **2** is an electronic communication device (cell phone, multimedia player, PDA or mobile computer), having built therein a wireless transmitter and receiver module **21** for communication with the wireless transmitter and receiver module **13** of the aforesaid emotion measuring finger cot unit **1,** a control unit **22** electrically connected with the wireless transmitter and receiver module **21,** and an input/output unit **23** electrically connected to the control unit **22.** The input/output unit **23** is operable by a medical practitioner (or the wearer) to input settings into the control unit **2** or to modify data in the control unit **2.** The input/output unit **23** can be incorporated with, for example, a LCD touchscreen display, keyboard or data transmission interface. The remote control device **2** can be connected to the internet or a remote server through a communication system so that a remote medical practitioner can obtain the measured finger skin temperature data from the emotion measuring finger cot unit **1** and then control and manage the settings (setting of the timer, setting of the standard time, etc.) of the measurement through the remote control device **2.** A remote medical practitioner can also give an advice to the wearer or to perform an on-line therapy via the remote control device **2** when obtained the wearer's finger skin temperature data.

Referring to FIG. 2, the measuring and remote control operation of the emotion measuring finger cot unit **1** and remote control device **2** of the medical system of the present invention are described hereinafter:
1. By means of the input/output unit **23** of the remote control device **2** the medical practitioner inputs a command of measurement to the control unit **22** so that the control unit **22** drives the wireless transmitter and receiver module **21** to send the command to the wireless transmitter and receiver module **13** of the aforesaid emotion measuring finger cot unit 1 wirelessly, and the wireless transmitter and receiver module **13,** immediately upon receipt of the command, transmits the command to the processor **12** for processing, and therefore the processor **12** controls the temperature sensor **11** to run subject to the received command;
2. When the emotion measuring finger cot unit **1** is worn on a finger of the wearer, the temperature sensor **11** is kept in contact with the skin of the wearer's finger to measure the skin temperature of the wearer's finger and to provide the measured skin temperature signal to the processor **12** for processing;
3. The processor **12** drives the wireless transmitter and receiver module **13** to transmit the processed skin temperature data to the wireless transmitter and receiver module **21** of the remote control device **2** wirelessly, enabling the wireless transmitter and receiver module **21** to load the received skin temperature data into the control unit **22;**
4. Upon receipt of the skin temperature data, the control unit **22** sends the skin temperature data to the input/output unit **23** for display, and therefore the medical practitioner (or the wearer) knows the emotional status of the wearer or the response of the wearer to a stress and can use the input/output unit **23** to record or monitor the skin temperature data; and
5. The medical practitioner can directly give the wearer a metal therapy to relieve emotional stress.

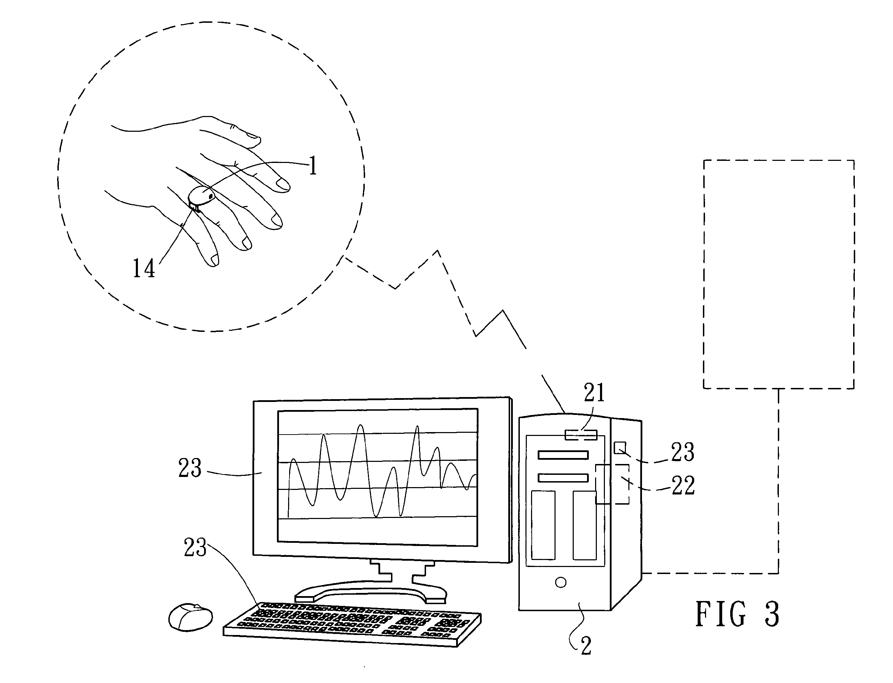

Further, as shown in FIG. 3, the control unit **22** can control the settings of the measurement of the emotion measuring finger cot unit **1.** Alternatively, the input/output unit **23** can be connected to the internet or a remote server through a communication system so that a remote medical practitioner can obtain the wearer's finger temperature data from the emotion measuring finger cot unit **1** at any time when desired and, control and manage the measuring operation (for example, the measuring time) through the remote control device **2,** and give a therapy or suggestion to help the wearer relieve the stress.

In actual application, the finger cot **14** is worn on a finger of the wearer and adjusted to the get the best fit, enabling the temperature sensor **11** to detect the skin temperature of the finger of the wearer accurately so that the processor **12** can obtain the temperature data accurately.

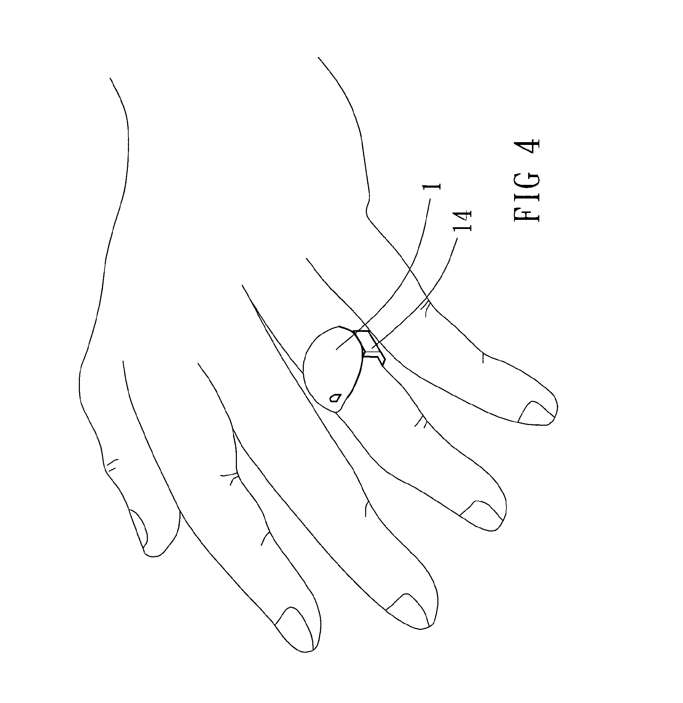

Referring to FIG. 4, the processor **12** and the wireless transmitter and receiver module **13** are built in the emotion measuring finger cot unit **1** for communication with the remote control device **2** wirelessly. Therefore, the emotion measuring finger cot unit **1** has simple structure and small size characteristics, suitable for wearing on a short finger of an adult or a finger of a child.

Further, the invention enables a medical practitioner to control the settings of the emotion measuring finger cot unit **1** and to obtain the measured data from the emotion measuring finger cot unit 1 through the remote control device **2.** Therefore, the medical practitioner can effectively monitor the emotional condition of the wearer and gives a proper control or therapy without facing the wearer, saving much time and medical cost and providing a good treatment effect. The medical system can also be used with the Internet or a communication system and a remote server so that an on-line metal therapy can be employed to help the wearer relieve emotional stress. Therefore, the use of the present invention avoids waste of medical resources and helps achieving substantial effects of medical popularization.

Although a particular embodiment of the invention has been described in detail for purposes of illustration, various modifications and enhancements may be made without departing from the spirit and scope of the invention. Accordingly, the invention is not to be limited except as by the appended claims.

## Claims

1. A medical system, comprising:
an emotion measuring finger cot unit **(1),** said emotion measuring finger cot unit **(1)** comprising a finger cot **(14)** wearable on a finger of a person, a temperature sensor **(11)** mounted in said finger cot **(14)** and adapted for measuring the skin temperature of the finger wearing said finger cot **(14),** a processor **(12)** electrically connected with said temperature sensor **(11)** and adapted for computing the finger skin temperature signal obtained through said temperature sensor **(11),** and a wireless transmitter and receiver module **(13)** electrically connected to said processor **(12)** and controllable by said processor **(12)** to transmit the finger temperature data to a remote control device **(2)** wirelessly; and
a remote control device **(2)** being an electronic communication device, said remote control device **(2)** comprising a wireless transmitter and receiver module **(21)** for communication with the wireless transmitter and receiver module **(13)** of said emotion measuring finger cot unit **(1),** a control unit **(22)** electrically connected with the wireless transmitter and receiver module **(21)** of said remote control device **(2),** and an input/output unit **(23)** electrically connected to said control unit **(22)** and operable by a person to input settings into said control unit **(22)** or to modify data in said control unit **(22).**

2. The medical system as claimed in claim 1, wherein said electronic communication device **(2)** is selected from the group of cell phone, multimedia player, PDA and computer.

3. The medical system as claimed in claim 1, wherein said processor **(12)** comprises data storage means built therein for storing the computed finger skin temperature data.

4. The medical system as claimed in claim 1, wherein said input/output unit **(23)** is incorporated with a LCD touchscreen display, keyboard or data transmission interface means for signal input.

5. The medical system as claimed in claim 1, wherein said input/output unit **(23)** of said remote control device **(2)** has means connectable to the internet for enabling a medical practitioner to monitor the measuring of said emotion measuring finger cot unit **(1)** and to employ a therapy at a remote location.

6. The medical system as claimed in claim 1, wherein said input/output unit **(23)** of said remote control device **(2)** has means connectable to a remote server through a communication system for enabling a medical practitioner to monitor the measuring of said emotion measuring finger cot unit **(1)** and to perform an on-line diagnosis.

7. The medical system as claimed in claim 1, wherein said finger cot **(14)** is a flexible member adjustable to fit the surface of the finger wearing said finger cot **(14).**
